Europäisches Patentamt

European Patent Office

Office européen des brevets

(19)

(11) Publication number: **0 076 665**
**B1**

(12) **EUROPEAN PATENT SPECIFICATION**

(45) Date of publication of patent specification: **14.01.87**

(51) Int. Cl.⁴: **A 61 M 1/34**

(21) Application number: **82305238.6**

(22) Date of filing: **01.10.82**

(54) Method and apparatus for plasmapheresis.

(30) Priority: **02.10.81 US 308055**

(43) Date of publication of application:
**13.04.83 Bulletin 83/15**

(45) Publication of the grant of the patent:
**14.01.87 Bulletin 87/03**

(84) Designated Contracting States:
**AT BE CH DE FR GB IT LI LU NL SE**

(56) References cited:
**DE-A-3 043 682**
**GB-A-1 381 410**
**US-A-4 191 182**

(73) Proprietor: **E.I. DU PONT DE NEMOURS AND COMPANY**
**1007 Market Street**
**Wilmington Delaware 19898 (US)**

(72) Inventor: **Ramstack, Joseph Michael**
**946 North Hill Drive**
**West Chester Pennsylvania 19380 (US)**

(74) Representative: **Jones, Alan John et al**
**CARPMAELS & RANSFORD 43 Bloomsbury Square**
**London, WC1A 2RA (GB)**

Courier Press, Leamington Spa, England.

## Description

Field of the invention
This invention relates to plasmapheresis by filtration.

Background information
Plasmapheresis is a process of separating plasma from whole blood. The plasma-depleted blood is comprised principally of cellular components, i.e., red blood cells, white blood cells and platelets. Plasma is comprised largely of water, but also contains proteins and various other noncellular compounds, both organic and inorganic.

Continuous plasmapheresis is a process of continuously removing whole blood from a subject, separating plasma from the blood and returning the plasma-depleted blood to the subject in a continuous extracorporeal circuit.

Plasmapheresis is currently used to obtain plasma for various transfusion needs, for preparation of fresh-frozen plasma, for subsequent fractionation to obtain specific proteins such as serum albumin, to produce cell culture media, and for disease therapies involving either the replacement of plasma or removal of specific disease-contributing factors from the plasma.

Plasmapheresis can be carried out by centrifugation or by filtration. Generally, in known filtration apparatus, while blood is conducted in a laminar flow path across one surface, i.e., the blood side surface, of a micro-porous membrane filter. Useful micro-porous membrane filters have pores which substantially retain the cellular components of blood but allow plasma to pass through. Such pores are referred to herein as cell-retaining pores. Typically, cell-retaining pore diameters are 0.1 µm to 1.0 µm.

Various filtration devices for phasmapheresis are disclosed in the literature. U.S. 3,705,100 discloses a center-fed circular membrane having a spiral flow path. U.S. 4,212,743 discloses a device having divergent flow channels. German Patent 2,925,143 discloses a filtration apparatus having parallel blood flow paths on one side of a membrane and parallel plasma flow paths, which are perpendicular to the blood flow paths, on the opposite surface of the membrane. U.K. Patent Application 2,037,614 discloses a rectilinear double-membrane envelope in which the membranes are sealed together at the ends of the blood flow path. U.K. Patent Specification 1,555,389 discloses a circular, center-fed, double-membrane envelope in which the membranes are sealed around their peripheries. German Patent 2,653,875 discloses a circular, center-fed double-membrane device in which blood flows through slot-shaped filter chambers.

DE—A—3 043 682 discloses a device for the separation of blood plasma, consisting of a membrane separator working together with a transmembrane pressure regulator for the flow of blood. The regulator maintains the desired transmembrane pressure.

US—A—4 191 182 discloses a process and apparatus for continuously separating blood into plasma and cellular component fractions and returning the latter to the subject in admixture with a makeup fluid. The separation is effected by continuously ultrafiltering the subject's blood at specified shear stresses and pressures employing a membrane ultrafilter, preferably have a pore size of 0.45 µm, and a disclosed flow system.

During plasmapheresis, it is desirable to attain high plasma separation efficiency. High plasma separation efficiency means that a large percentage of available plasma is removed. Membrane fouling, however, may impede maintenance of high separation efficiency for an extended period of time. Membrane fouling is discussed in Asanuma, Y., et al., *Proc. Euro. Soc. Artif. Organs 6*: 308, 1979 and Folstrom, R. J., et al., *Trans. Am. Soc. Artif. Organs 21*: 602, 1975. It is believed to be a response to convective forces depositing components of the blood on the membrane. As fouling progresses, plasma flow will decline or can be maintained by increasing the difference in pressure between the blood side and plasma side of the membrane, i.e., transmembrane pressure difference. High transmembrane pressure difference may cause undesirable molecular scale sieving and blood trauma.

It is an object of this invention to provide a method for plasmapheresis by filtration which can be carried out with high plasma separation efficiency for long periods of time by passing plasma through a membrane in an efficient manner, and apparatus therefor.

Disclosure of the invention
For further comprehension of the invention and of the objects and advantages thereof, reference may be had to the following description and to the appended claims in which the various novel features of the invention are more particularly set forth.

The invention resides in the discovery that plasmapheresis by filtration through a membrane can be carried out with high plasma separation efficiency for long periods of time by conducting blood over a surface of a membrane and, in a cyclic manner, (a) passing plasma through the membrane and, when the ceiling system transmembrane pressure difference is attained, (b) reducing the flow of plasma through the membrane and reducing system transmembrane pressure difference (STMP), preferably to about zero.

The invention also resides in apparatus for carrying out plasmapheresis by filtration through a membrane which comprises a membrane, means for conducting blood over a surface of the membrane and means for passing plasma through the membrane, and means for reducing the flow of plasma through the membrane and reducing STMP, preferably to about zero, when the ceiling STMP is attained.

In its preferred embodiment, the invention resides in said process which comprises passing plasma at an accelerating rate until the threshold level is attained and in said process which further comprises reducing the rate of acceleration preferably to about zero and, when the ceiling STMP is attained, substantially terminating the passing of plasma and reducing STMP to about zero, and in apparatus comprising means for carrying out these steps.

The invention may be best understood by reference to the following illustrative figures.

Brief description of the drawings

Figure 1 is a flow diagram of the preferred embodiment of the invention.

Figure 2a is a plot of system transmembrane pressure difference versus time.

Figure 2b is a plot of plasma flow rate versus time and is superimposable upon Figure 2a.

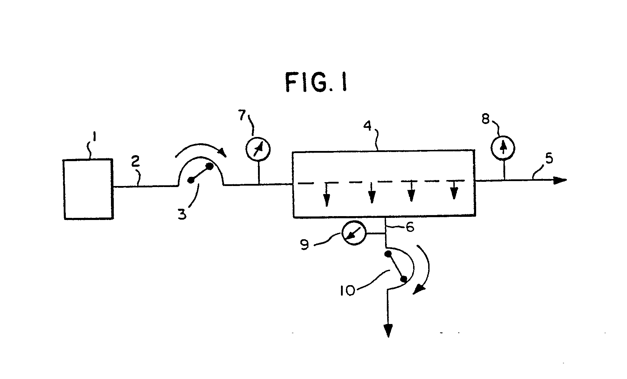

Referring to Figure 1, which illustrates the preferred scheme for carrying out the invention, blood is continuously conducted from a source 1, e.g., human donor or patient through a blood line 2, by means of a pump 3 into plasmapheresis membrane filter module 4. Within module 4, plasma is separated from the blood by filtration. Plasma-depleted blood exits from the module via line 5 while plasma exits via line 6. Pressure transducers 7, 8, 9 are located near the inlet to the module on line 2, near the plasma-depleted blood outlet on line 5 and near the plasma outlet on line 6, respectively. A plasma pump 10, draws plasma through the membrane in the module by pumping plasma away from the membrane.

Pressure transducers 7, 8, 9 are used to monitor STMP which is defined as follows:

$$[(\text{blood inlet pressure} + \text{blood outlet pressure}) \div 2]$$
$$- \text{plasma outlet pressure.}$$

The modifier, "system", is used to indicate that the transmembrane pressure difference being monitored is the average across the entire module and not the transmembrane pressure difference at any one point on a membrane within the module.

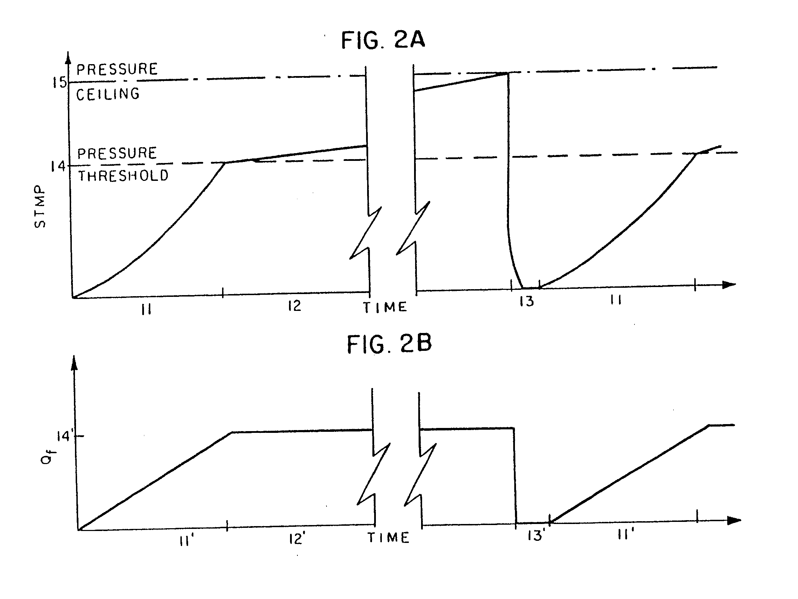

The preferred manner of passing plasma through the membrane and reducing the passing of plasma and reducing STMP is illustrated by Figures 2a and 2b. Referring to Figure 2a, plasma is initially drawn at a slow rate. The rate of drawing is accelerated during time period 11, e.g., 5 ml-min$^{-1}$-min$^{-1}$, providing an accelerating increase in STMP. This acceleration is continued, typically for less than about 5 minutes to about 10 minutes, until the STMP reaches the threshold level 14, e.g., about 50 mm Hg (6.7 kPa), after which the rate of acceleration is reduced to zero, i.e., the plasma flow rate ($Q_f$) is maintained constant, during time period 12. Despite the constant $Q_f$, STMP continues to rise. When STMP reaches ceiling pressure 15, e.g., about 100 mm Hg (13.3 kPa), the plasma pump is turned off and STMP is thereby reduced to about zero, allowing the membrane to be cleared during time period 13, typically less than about 5 minutes. Membrane clearing during time period 13 is enhanced by the use of fouling-reducing techniques while conducting the blood over the surface of the membrane, e.g., reciprocatory pulsatile flow, high blood velocity and blood recycle.

Reciprocatory pulsatile flow is the preferred manner of conducting blood over the membrane in carrying out this invention, although it is to be noted that plasmapheresis by filtration using reciprocatory pulsatile flow is the invention of another. It comprises, in summary, oscillating blood in a flow path on a surface of a membrane with a net movement of blood from inlet to outlet of the flowpath while collecting plasma which passes through the membrane. Means for carrying out this process include, e.g., a plurality of coordinated pumps and valves positioned on blood inlet, plasma-depleted blood outlet and plasma lines, pressure accumulators, or surge chambers, may also be useful. Blood recycle is a means of achieving high blood velocity which has also been shown to reduce membrane fouling.

In a preferred apparatus for carrying out the invention, the $Q_f$ is controlled electronically in response to the STMP, e.g., an electronic device such as a microprocessor which automatically accelerates $Q_f$, ceases the acceleration when the threshold level is attained, reduces $Q_f$ and STMP when the ceiling pressure is attained and repeats the cycle after a fixed duration. The threshold level is the STMP or rate of STMP increase which provides the optimal plasma separation efficiency, i.e., high $Q_f$ with a moderate rate of STMP increase without unacceptable sieving and blood trauma. If the rate of passing plasma is accelerated beyond the optimal threshold level, a very high $Q_f$ may be achieved, but STMP will rapidly increase to the ceiling pressure resulting in a short time period during which plasma is separated and, possibly, in irreversible fouling. If the rate of passing plasma does not attain the threshold level, or does so too slowly, $Q_f$ will be undesirably low even though the ceiling STMP may be reached very slowly. The ceiling pressure is the pressure above which unacceptable fouling and blood trauma begin to occur.

Referring to Figure 2b, $Q_f$ increases during time period 11' until the threshold level 14' is attained, after which, during time period 12', $Q_f$ is substantially constant. When STMP is reduced, $Q_f$ substantially ceases, i.e., during time period 13'. $Q_f$ resumes when the cycle is repeated.

It should be emphasized that the above description is an example only. The specified $Q_f$ acceleration rate, threshold level and ceiling pressure may not be optimal. They are typical of a plasmapheresis treatment allowing for a substantial margin of safety in avoiding blood trauma while providing acceptable plasma separation efficiency. The optimal values in a particular case may vary with several factors, e.g., module design, manner of conducting the blood over the membrane and characteristics of the blood. Further, while it is believed to be preferable to substantially terminate $Q_f$ and reduce STMP to about zero when the ceiling STMP is attained, reversing the flow of plasma or reducing STMP to below zero is not precluded, just as other variations not specifically discussed herein, e.g., irregular acceleration of $Q_f$, are not precluded.

While the preferred embodiments of the invention are described above, it is to be understood that the invention is not limited to the precise embodiments herein disclosed and that the right to all changes and modifications coming within the scope of the invention as defined in the following claims is reserved.

## Claims

1. A process for plasmapheresis by filtration which comprises conducting blood over the first surface of a microporous membrane having first and second surfaces, while collecting plasma-depleted blood from the first surface and plasma which flows through the membrane (plasma flow) from the second surface, wherein during the process the system transmembrane pressure (STMP) reaches a ceiling, characterized in that when the ceiling STMP is attained, the plasma flow and STMP are reduced to about zero, and thereafter plasma flow and STMP are increased and the collection of plasma is continued, with the proviso that said plasma and said plasma-depleted blood are not returned to the donor body from which the blood is drawn.

2. Process of Claim 1 which further comprises conducting blood over the surface of the membrane using fouling-reducing techniques.

3. Process of Claim 1 or Claim 2 which further comprises conducting blood over the surface of the membrane by reciprocatory pulsatile flow.

4. Process of any one of Claims 1 to 3 which comprises passing plasma at an accelerating rate until the threshold level is attained.

5. Process of any one of Claims 1 to 4 which further comprises electronically controlling the plasma flow in response to the system transmembrane pressure.

6. Apparatus for carrying out plasmapheresis by filtration which comprises means (2, 3, 4) for conducting blood over the first surface of a microporous membrane having first and second surfaces, means (5, 6, 10) for collecting plasma-depleted blood from the first surface and plasma which flows through the membrane (plasma flow) from the second surface, means (7, 8, 9) for measuring system transmembrane pressure (STMP), and means for reducing plasma flow and STMP to about zero.

7. Apparatus of Claim 6 which includes means for conducting blood over the surface of the membrane using fouling-reducing techniques.

8. Apparatus of Claim 6 or Claim 7 which includes means for conducting blood over the surface of the membrane by reciprocatory pulsatile flow.

9. Apparatus of any one of Claims 6 to 8 which includes means for passing plasma at an accelerating rate until the threshold level is attained.

10. Apparatus of any one of Claims 6 to 9 which includes an electronic device which controls the plasma flow in response to the system transmembrane pressure.

## Patentansprüche

1. Verfahren zur Plasmapherese durch Filtration, in dem Blut über die erste Oberfläche einer mikroporösen Membran mit einer ersten und einer zweiten Oberfläche geleitet wird, während das an Plasma verarmte Blut von der ersten Oberfläche und das Plasma, das durch die Membran hindurch fließt (Plasmafluß), von der zweiten Oberfläche gesammelt wird, wobei während des Verfahrens der System-Trans-Membran-Druck (STMP) einen oberen Grenzwert erreicht, dadurch gekennzeichnet, daß bei Erreichen des oberen STMP-Grenzwertes der Plasmafluß und der STMP auf etwa Null reduziert werden und danach der Plasmafluß und der STMP erhöht werden und das Sammeln des Plasmas fortgesetzt wird, mit der Maßgabe, daß das Plasma und das an Plasma verarmte Blut nicht dem Körper des Spenders zurückgegeben werden, dem das Blut abgenommen wird.

2. Verfahren nach Anspruch 1, dadurch gekennzeichnet, daß es weiterhin das Leiten von Blut über die Oberfläche der Membran unter Einsatz von Techniken der Verringerung der Verschmutzung umfaßt.

3. Verfahren nach Anspruch 1 oder Anspruch 2, dadurch gekennzeichnet, daß es weiterhin das Leiten von Blut über die Oberfläche der Membran mittels eines hin- und herbewegten pulsierenden Flusses umfaßt.

4. Verfahren nach irgendeinem der Ansprüche 1 bis 3, dadurch gekennzeichnet, daß das Plasma mit einer beschleunigten Geschwindigkeit geleitet wird, bis der Schwellenwert erreicht ist.

5. Verfahren nach irgendeinem der Ansprüche 1 bis 4, dadurch gekennzeichnet, daß es weiterhin die elektronische Steuerung des Plasmaflusses als Antwort auf den System-Trans-Membran-Druck umfaßt.

6. Apparat zur Durchführung der Plasmapherese durch Filtration, der Mittel (2, 3, 4) zum Leiten des Blutes über die erste Oberfläche einer mikroporösen Membran mit einer ersten und einer zweiten Oberfläche, Mittel (5, 6, 10) zum Sammeln des an Plasma verarmten Blutes von der ersten Oberfläche und des Plasmas, das durch die Membran hindurch fließt (Plasmafluß), von der zweiten Oberfläche, Mittel (7, 8, 9) zum Messen des System-Trans-Membran-Druckes (STMP) sowie Mittel zur Verringerung des Plasmaflusses und des STMP auf etwa Null umfaßt.

7. Apparat nach Anspruch 6, der Mittel zum Leiten von Blut über die Oberfläche der Membran unter Einsatz von Techniken der Verringerung der Verschmutzung umfaßt.

8. Apparat nach Anspruch 6 oder Anspruch 7, der Mittel zum Leiten von Blut über die Oberfläche der Membran mittels eines hin- und herbewegten pulsierenden Flusses umfaßt.

9. Apparat nach irgendeinem der Ansprüche 6 bis 8, der Mittel zum Leiten des Plasmas mit einer beschleunigten Geschwindigkeit umfaßt, bis der Schwellenwert erreicht ist.

10. Apparat nach irgendeinem der Ansprüche 6 bis 9, der eine elektronische Vorrichtung umfaßt, die den Plasmafluß als Antwort auf den System-Trans-Membran-Druck steuert.

**Revendications**

1. Procédé de plasmaphérèse par filtration, qui consiste à conduire le sang sur la première surface d'une membrane microporeuse ayant une première et une deuxième surfaces, tout en recueillant sur la première surface du sang privé de plasma et sur la deuxième surface, du plasma qui s'écoule à travers la membrane (écoulement de plasma), et dans lequel, pendant le processus, la pression transmembrane du système (STMP) atteint un plafond, caractérisé par le fait que lorsque le plafond de STMP est atteint, on réduit l'écoulement de plasma et la STMP aux environs de zéro et ensuite, on augmente l'écoulement de plasma et la STMP et on continue de recueillir le plasma, étant entendu que le plasma et le sang privé de plasma ne sont pas restitués à l'organisme du donneur duquel le sang est tiré.

2. Procédé selon la revendication 1, qui consiste en outre à conduire du sang sur la surface de la membrane en utilisant des techniques de diminution d'encrassement.

3. Procédé selon la revendication 1 ou la revendication 2, qui consiste en outre à conduire le sang sur la surface de la membrane par écoulement pulsatile alterné.

4. Procédé selon l'une quelconque des revendications 1 à 3, qui consiste à faire passer le plasma à une vitesse accélérée jusqu'à ce que le niveau de seuil soit atteint.

5. Procédé selon l'une quelconque des revendications 1 à 4, qui consiste en outre à commander électroniquement l'écoulement de plasma en réponse à la pression transmembrane du système.

6. Appareil pour l'exécution de la plasmaphérèse par filtration, qui comprend des moyens (2, 3, 4) pour conduire le sang sur la première surface d'une membrane microporeuse ayant une première et une deuxième surfaces, des moyens (5, 6, 10) pour recueillir sur la première surface du sang privé de plasma et sur la deuxième surface du plasma qui s'écoule à travers la membrane (écoulement de plasma), des moyens (7, 8, 9) pour mesurer la pression transmembrane du système (STMP) et des moyens pour réduire l'écoulement de plasma et la STMP aux environs de zéro.

7. Appareil selon la revendication 6, qui comprend des moyens pour conduire le sang sur la surface de la membrane en utilisant des techniques de diminution d'encrassement.

8. Appareil selon la revendication 6 ou la revendication 7, qui comprend des moyens pour conduire le sang sur la surface de la membrane par écoulement pulsatile réciproque.

9. Appareil selon l'une quelconque des revendications 6 à 8, qui comprend des moyens pour faire passer du plasma à une vitesse accélérée jusqu'à ce que le niveau de seuil soit atteint.

10. Appareil selon l'une quelconque des revendications 6 à 9, qui comprend un dispositif électronique qui commande l'écoulement du plasma en réponse à la pression transmembrane du système.

# FIG. 1

## FIG. 2A

PRESSURE
CEILING

PRESSURE
THRESHOLD

STMP

15

14

I1    I2    TIME    I3    I1

## FIG. 2B

$Q_f$

14'

I1'    I2'    TIME    I3'    I1'